# EUROPEAN PATENT SPECIFICATION

(11) **EP 1 898 842 B1**
(45) Date of publication and mention of the grant of the patent: **27.11.2013**
(21) Application number: 06792457.1
(22) Date of filing: 28.04.2006
(51) Int. Cl.: A61F 2/30

(54) **PROSTHETIC COMPONENT WITH RECESSES PROVIDED BENEATH THE OUTER SURFACE**
PROTHESENKOMPONENTE MIT AUSSPARUNGEN UNTER DER ÄUSSEREN OBERFLÄCHE
ÉLÉMENT DE PROTHÈSE COMPORTANT DES ÉVIDEMENTS FORMÉS SOUS LA SURFACE EXTERIEURE

(30) Priority: 02.05.2005 CH 7672005
(43) Date of publication of application: 19.03.2008
(73) Proprietor: Adler Ortho S.r.l., 20032 Cormano (IT)
(72) Inventor: RAGBIR, Sheila, Whim (TT)
(74) Representative: Modiano, Micaela Nadia
(86) International application number: PCT/EP2006/061937
(87) International publication number: WO 2006/125711

(56) References cited:
- DE-A1- 19 614 949
- US-A- 5 021 063
- US-A- 5 108 435
- US-A- 6 127 596

## Description

The present invention relates to the technological field of prostheses for orthopedic or traumatologic surgery, such as for example hip prostheses, shoulder prostheses, et cetera.

More particularly, the invention relates to the sector of the technological field that deals with the design and manufacture of components that constitute prostheses, which are inserted in contact with bone parts which subsequently, by regrowing after surgery, incorporate them and remain rigidly coupled thereto.

In order to achieve this result, said prosthetic components are provided so that the part of their surface that makes contact with the bone is as furrowed and porous as possible, so that the trabeculae of the bone tissue that regenerates wedge within the unevennesses of the surface of the prosthetic components, gripping them and applying a reliable coupling action.

Unfortunately, what has been described above does not always occur, or doe not always occur in a sufficiently reliable manner, when using the prosthetic components known in the background art, and the resulting connection between the bone and a prosthetic component is often insufficient to make them reliably and durably coupled in the presence of the intense torsional and/or flexural stresses to which the skeletal parts of the patient are subjected after surgery.

The inventor of the present invention has thought that in order to allow the bone, during its regrowth, to perform a reliable coupling action on the prosthesis, it is necessary to provide the components that constitute said prosthesis and make contact with bone parts so that they have recesses which are closed and covered externally, i.e., in the direction of the bone parts, by an abutment surface, below which the bone can penetrate during its regrowth, penetrating within said recesses through small holes formed in said abutment surface. The trabeculae of the regrowth bone tissue, by expanding within the recesses after passing through the above described holes until they occupy the entire volume of said recesses, provide a true anchoring action, which stably couples to the bone parts the prosthetic components that make contact with said bone parts.

In order to achieve this result, the inventor has devised prosthetic components in which, below the layer provided with their outer surface that makes contact with bone parts, there are multiple recesses, which are connected to the outside through openings which pass through the layer provided with said outer surface. In a preferred embodiment, the inventor provides for said recesses to be comprised between multiple rod-like elements of equal length, which are rigidly coupled, at their ends, respectively to the layer provided with the outer surface and to the body of the prosthetic component.

DE 19614949, US-5,021,063 and US-5,108,435 disclose a prosthesis as defined in the preamble of claim 1.

US-6,127,596 discloses a prosthesis having a grid-like surface.

The present invention therefore relates to a prosthetic component as described in the accompanying claim 1.

A more detailed description of a preferred example of embodiment of an element (a cotyloid element for a hip prosthesis) provided according to the invention is now given. During said description, reference will be made also to the accompanying drawings, wherein:

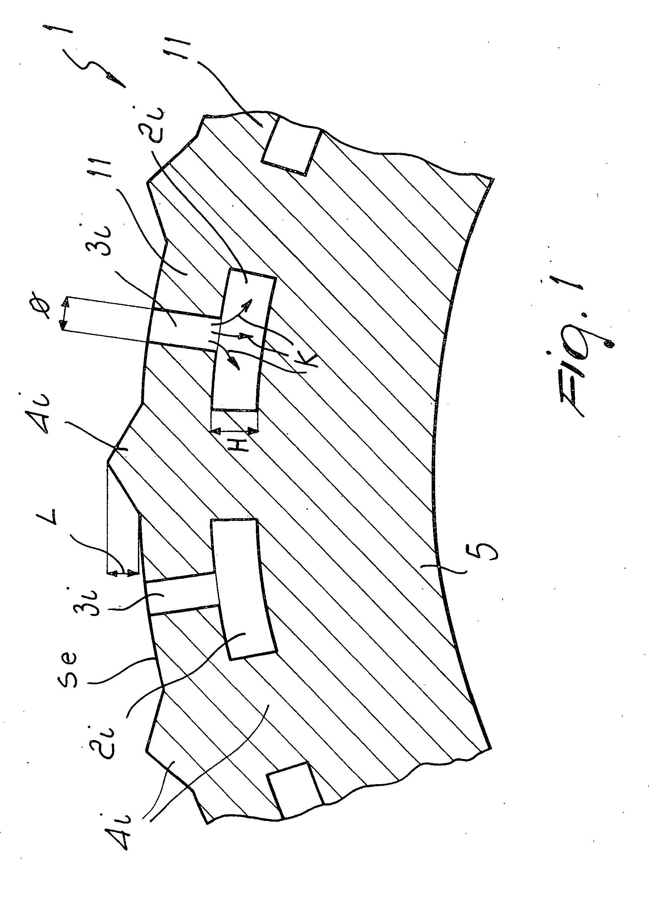

Figure 1 is a highly enlarged-scale longitudinal sectional view of a portion of a prosthetic component according to the invention;
Figure 2 is an enlarged-scale side view of the prosthetic component;
Figure 3 is a likewise enlarged-scale longitudinal sectional view;

With reference to the figures, the reference numeral 1 designates a prosthetic component according to the invention (a cotyloid element for a hip prosthesis, in the case being described), which has an outer surface Se which is meant to make contact, and remain in contact, with bone parts after a surgical procedure.

Below a layer 11 provided with said outer surface Se there are multiple mutually adjacent recesses 2i, which are comprised between the layer 11 and the body 5 of the component 1 and are delimited by rod-like elements 4i (which might also be partitions - a case which is not shown), the ends of which are rigidly coupled respectively to the above described layer 11 and to the body of the prosthetic component 1. Of course, the height of the rod-like elements 4i below the layer 11 is equal to the height H of the recesses 2i (said height H can be comprised discretionally for example between 0.15 and 0.6 mm). The recesses 2i can therefore be connected or not to each other along their perimeter, depending on whether they are delimited by the rod-like elements (as in the figures) or by the partitions mentioned above.

The recesses 2i are connected to the outside (i.e., toward the bone parts that makes contact with the outer surface Se of the component 1), by virtue of openings 3i (preferably one for each recess 2i), which pass through the layer 11 provided with the outer surface Se. In the case being considered, said openings are, for example, circular holes 3i having a diameter 0 or holes having another shape.

The bone trabeculae, in their regrowth, after passing through the holes 3i, fill the volume of each recess 2i (see the arrows K), forming bodies which can no longer separate from the component 1 by exiting through the holes 3i. A reliable and durable anchoring is therefore produced. In order to improve the adhesion between the outer surface Se of an element 1 according to the invention and the surrounding bone parts, the rod-like elements 4i can be provided so that they protrude by a preset height L with respect to the outer surface Se of the prosthetic component 1.

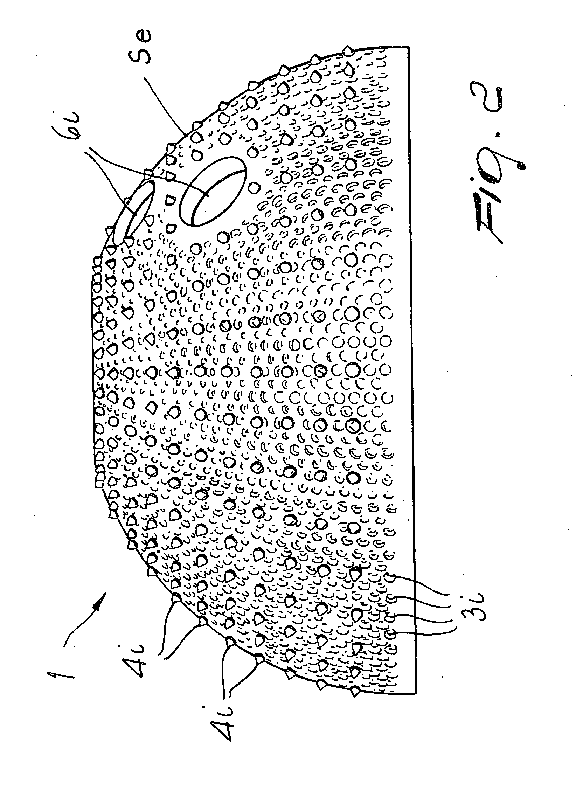

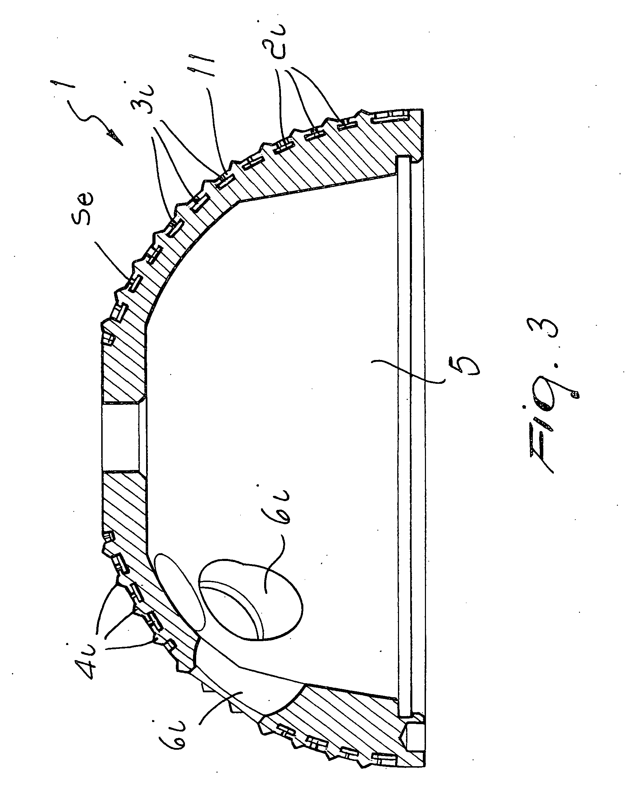

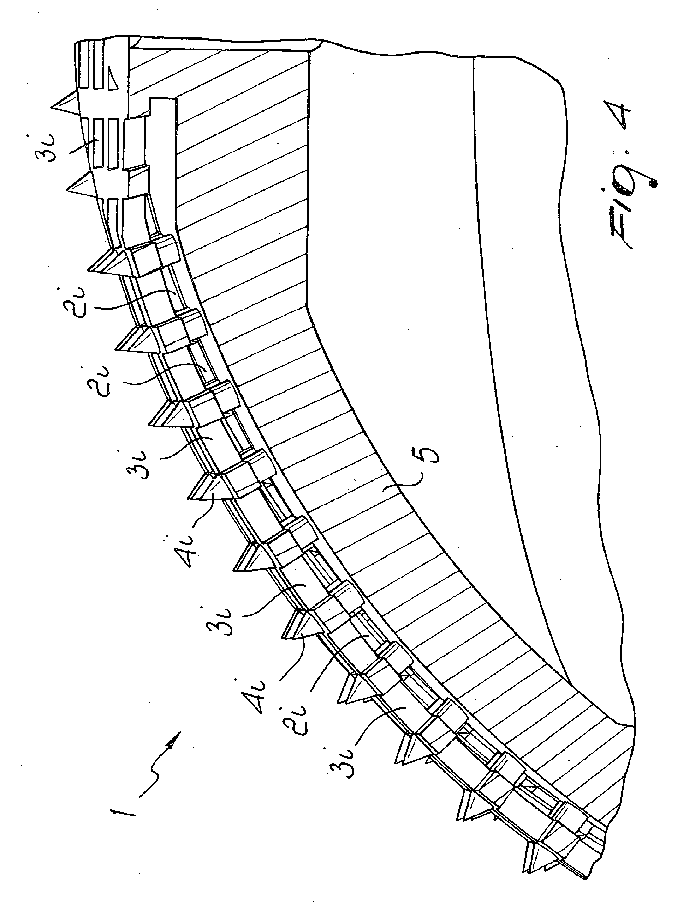

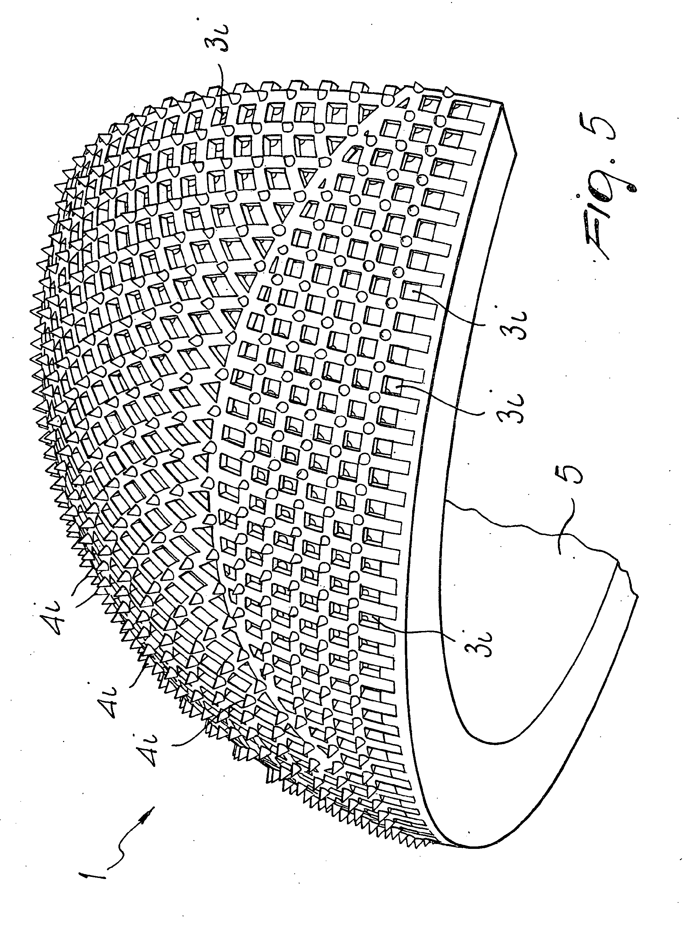

Figures 2 and 3 show in full a prosthetic component 1 provided as described up to now. Said Figures show that the possibility has also been provided of forming one or more threaded through holes 6i, which pass both through the layer 11 provided with the outer surface Se and through the body 5 of the component 1. In the cases of the application of a hip prosthesis, this solution allows to apply optionally other auxiliary fixing elements (not shown) if their action is required before completing the regrowth process of the bone parts or for other reasons. If said holes 6i are not needed, or are no longer needed, they can be closed by means of a corresponding number of threaded plugs (also not shown in the drawings).

Conveniently, the recesses 2i of the prosthetic component 1 forming a cotyloid element are arranged along meridians and parallels, which can be traced ideally on the outer surface Se of the hemisphere of the prosthetic component 1.

A prosthetic component according to the invention is entirely made of a titanium alloy by using the technique of sintering by means of an electron beam (EBM, electron beam melting), which can be used not only for forming complex synthetic resin structures but also for structures made of other metallic alloys.

Starting from this alloy in powder form, and by performing an electron beam melting process by means of the successive melting of the points that lie at the intersection of two electron beams, it is possible to provide easily a prosthetic component according to the invention. The electron beams must of course be driven by means of a computer which runs a program suitable for this purpose.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A prosthetic component (1) for orthopedic or traumatologic surgery meant to be inserted in a bone part, wherein below a layer (11) which has an outer surface (Se) meant to make contact with a bone part there are multiple recesses (2i), which are connected to the outside through openings (3i) which pass through said layer (11) provided with said outer surface, said recesses (2i) being comprised between multiple rod-like elements (4i) of equal height, which are rigidly coupled, at their ends, to said outer surface (Se) and to the body (5) of said component (1), said rod-like elements (4i) protruding outside of said outer surface by a preset height (L), **characterized in that** the prosthetic component (1) is provided with one or more threaded through holes (6i), which cross both the layer (11) provided with said outer surface (Se) and the body (5) of said component (1).

2. The prosthetic component of claim 1, **characterized in that** it is made of an alloy of titanium or of other metals.

## Patentansprüche

1. Eine prothetische Komponente (1) für die orthopädische oder traumatologische Chirurgie, die dazu bestimmt ist, in ein Knochenteil eingesetzt zu werden, wobei sich unterhalb einer Schicht (11), die eine äußere Oberfläche (Se) hat, welche dazu bestimmt ist, in Kontakt mit einem Knochenteil zu stehen, mehrere Vertiefungen (2i) befinden, die mit der Außenseite durch Öffnungen (3i) verbunden sind, welche durch die Schicht (11) verlaufen, die mit der äußeren Oberfläche versehen ist, wobei die Vertiefungen (2i) sich zwischen mehreren stabartigen Elementen (4i) gleicher Höhe befinden, welche an ihren Enden starr mit der äußeren Oberfläche (Se) und mit dem Körper (5) der Komponente (1) verbunden sind, wobei die stabartigen Elemente (4i) mit einer vordefinierten Höhe (L) aus der äußeren Oberfläche herausragen, **dadurch gekennzeichnet, dass** die prothetische Komponente (1) mit einer oder mehreren, mit einem Gewinde versehenen, durchgehenden Bohrungen (6i) versehen ist, die sowohl die mit der äußeren Oberfläche (Se) versehene Schicht (11) als auch den Körper (5) der Komponente (1) durchqueren.

2. Die prothetische Komponente gemäß Anspruch 1, **dadurch gekennzeichnet, dass** sie aus einer Legierung von Titan oder von anderen Metallen besteht.

## Revendications

1. Elément de prothèse (1) pour la chirurgie orthopédique ou traumatologique destiné à être inséré dans une partie d'os, dans lequel, en dessous d'une couche (11) qui présente une surface externe (Se) destinée à entrer en contact avec une partie de l'os, il y a de multiples évidements (2i), qui sont reliés à l'extérieur par des ouvertures (3i) qui traversent, ladite couche (11) pourvue de ladite surface externe, lesdits évidements (2i) étant compris entre de multiples éléments en forme de tige (4i) de même hauteur, qui sont couplés rigidement, à leurs extrémités, à ladite surface externe (Se) et au corps (5) dudit élément (1), lesdits éléments en forme de tige (4i) faisant saillie à l'extérieur de ladite surface externe d'une hauteur prédéfinie (L), caractérisé en ce l'élément de prothèse (1) comporte un ou plusieurs trous traversants tarandés (6i), qui traversent à la fois la couche (11) pourvue de ladite surface externe (Se) et le corps (5) dudit élément (1).

2. Elément de prothèse selon la revendication 1, **caractérisé en ce que** il est constitué d'un alliage de titane ou d'autres métaux.
